# FASCICULE DE BREVET EUROPEEN

(11) **EP 4 447 881 B1**
(45) Date de publication et mention de la délivrance du brevet: **08.10.2025**
(21) Numéro de dépôt: 22839709.7
(22) Date de dépôt: 16.12.2022
(51) Int. Cl.: A61F 5/14, A61F 5/01

(54) **SEMELLE ORTHOPÉDIQUE TEXTURÉE**
STRUKTURIERTE ORTHOPÄDISCHE EINLEGESOHLE
TEXTURED ORTHOPAEDIC SOLE

(30) Priorité: 17.12.2021 EP 21306826
(43) Date de publication de la demande: 23.10.2024
(73) Titulaire: Med'Insole, 94410 Saint-Maurice (FR)
(72) Inventeur: GAIGHER, Isabelle, 94410 Saint-Maurice (FR); DIAS, Mickaël, 94410 Saint-Maurice (FR)
(74) Mandataire: Icosa
(86) Numéro de dépôt international: PCT/EP2022/086306
(87) Numéro de publication internationale: WO 2023/111245

(56) Documents cités:
- CA-A1- 3 121 925
- US-A1- 2010 205 831
- US-A1- 2021 161 248

## Description

### DOMAINE DE L'INVENTION

La présente invention concerne les semelles orthopédiques. En particulier, la présente invention concerne les semelles orthopédiques proprioceptives texturées.

### ÉTAT DE LA TECHNIQUE

La rétroaction sensorielle (« feed-back ») provenant de la plante du pied joue un rôle important dans le contrôle de l'équilibre. La diminution de la rétroaction sensorielle, due au vieillissement et/ ou à la maladie, a été étroitement associée à une mobilité réduite et aux chutes chez les personnes âgées, les personnes souffrant de pathologies neuro-dégénératives (maladie de Parkinson, Sclérose en plaque et maladie de Charcot Marie Tooth) ou les personnes souffrant de neuropathie périphérique (diabétiques ou suite à des accidents : AVC par exemple). Ces personnes présentent, de façon générale, une dégradation des sensations périphériques et ne savent plus contrôler ou corriger leur posture grâce aux informations leur provenant des mécanorécepteurs présents sur la face plantaire de leur pied.

Des semelles ayant pour but d'absorber les chocs et fournir un soutien aux zones du pied les plus soumises à une force ou une pression accrue pendant la station debout, la marche ou d'autres activités sont déjà connues, mais elles ne permettent pas d'améliorer la rétroaction sensorielle. Le document US 2010/205831 divulgue une telle semelle comprenant des zones d'amortissement sur le côté inférieur de la semelle, i.e. au contact de la chaussure. Ce document décrit une semelle orthopédique suivant le préambule de la revendication 1.

Il existe donc un besoin pour l'amélioration artificielle de la rétroaction sensorielle provenant de la plante du pied afin d'améliorer la perception de la posture debout et le contrôle de la posture.

La présente invention a donc pour objet de stimuler les mécanorécepteurs de la face plantaire du pied en fournissant une semelle orthopédique comprenant une structure alvéolaire de stimulation de la face plantaire du pied localisée sur une ou plusieurs zones précises contenant desdits mécanorécepteurs. La stimulation des mécanorécepteurs par la structure alvéolaire de stimulation est créée par le mouvement irrégulier dans les trois directions de l'espace des alvéoles lors de la marche ou en position orthostatique. Cette stimulation influence la modulation de la réponse posturale et permet un meilleur contrôle de l'équilibre, prévenant ainsi des chutes.

### RÉSUMÉ

La présente invention a pour objet une semelle orthopédique selon la revendication 1.

Dans un mode de réalisation, au moins une zone de stimulation est choisie parmi la première zone, la troisième zone ou la cinquième zone de la semelle. Dans un mode de réalisation, la semelle comprend au moins deux zones de stimulation choisies parmi la première zone, la troisième zone et/ou la cinquième zone de la semelle. Dans un mode de réalisation, la première zone, la troisième zone et la cinquième zone de la semelle sont des zones de stimulation. Dans un mode de réalisation, au moins une zone de stimulation comprend en outre des éléments de stimulation répartis dans les alvéoles de la première structure alvéolaire de stimulation. Dans un mode de réalisation, les éléments de stimulation ont une forme sphérique, hémisphérique, conique, parallélépipédique, polyédrique, pyramidale, ou cylindrique. Dans un mode de réalisation, la première zone et la cinquième zone de la semelle comprennent en outre des éléments de stimulation répartis dans les alvéoles de leur première structure alvéolaire de stimulation et dans laquelle la troisième zone de la semelle comprend une première structure alvéolaire de stimulation vide. Dans un mode de réalisation, au moins une zone de la semelle comprend une seconde structure alvéolaire de stimulation de la face plantaire du pied, ladite seconde structure alvéolaire de stimulation comprenant une pluralité d'alvéoles, chacune délimitée par au moins une paroi s'étendant transversalement à la face plantaire. Dans un mode de réalisation, la au moins une zone comprenant ladite seconde structure alvéolaire de stimulation est choisie parmi la deuxième zone ou la quatrième zone de la semelle. Dans un mode de réalisation, la deuxième zone et la quatrième zone de la semelle comprennent chacune une seconde structure alvéolaire de stimulation. Dans un mode de réalisation, la hauteur des parois des alvéoles de la seconde structure alvéolaire de stimulation est plus faible que celle des parois des alvéoles de la première structure alvéolaire de stimulation. Dans un mode de réalisation, la première structure alvéolaire de stimulation et/ou la seconde structure alvéolaire de stimulation a une structure en nid d'abeilles.

La présente invention a également pour objet un élément chaussant comprenant une semelle orthopédique selon l'invention.

La présente invention a également pour objet un procédé de fabrication d'une semelle orthopédique selon l'invention.

### DÉFINITIONS

Dans la présente invention, les termes ci-dessous sont définis de la manière suivante :
**"Anneau talonnier"** concerne anneau stabilisateur situé sur une semelle et entourant le talon. Il stabilise l'arrière pied et a un but purement mécanique. De préférence, l'anneau talonnier a une hauteur comprise entre 1 mm et 20 mm, plus préférentiellement entre 1 mm et 10 mm, encore plus préférentiellement entre 4 mm et 7 mm.
**"Face plantaire"** concerne la face de la semelle destinée à être mise en contact avec la plante du pied.
**"Semelle orthopédique"** concerne une semelle destinée à être placée dans un élément chaussant tel qu'une chaussure ou un chausson, et comprenant des éléments de stabilisation tels que, par exemple, un soutien de voûte plantaire et un anneau talonnier. La semelle orthopédique permet donc de stabiliser un sujet en évitant au pied d'osciller en interne et en externe. La semelle orthopédique stabilise donc surtout en interne et en externe, *i.e.* selon l'axe de prono-supination, et répartit de façon plus homogène les zones d'appui plantaire. La semelle orthopédique n'est pas solidaire de l'élément chaussant, il s'agit d'une semelle « libre » pouvant être placée dans n'importe quel élément chaussant. Cela a pour avantage de permettre à l'utilisateur d'utiliser une unique paire de semelles orthopédiques selon l'invention pour tous ses éléments chaussants.
**"Structure en nid d'abeilles"** concerne un réseau de cellules, ou alvéoles, creuses formées entre de minces parois verticales. Les cellules sont de forme hexagonale ayant une densité minimale et des propriétés de compression hors plan relativement élevées et des propriétés de cisaillement hors plan.
**"Soutien de voûte plantaire"** d'une semelle concerne une voûte artificielle, c'est-à-dire une arche au niveau des premier et deuxième métatarsiens et du tarse antéro-interne. Elle permet la stimulation de la pompe veineuse plantaire afin d'améliorer le retour veineux et la stabilisation du pied en prévenant son effondrement en interne, dit aussi en valgus.

### DESCRIPTION DÉTAILLÉE

### Semelle orthopédique

La présente invention concerne une semelle orthopédique selon la revendication 1.

En d'autres termes, ladite au moins une alvéole est perforée pour former un trou préférentiellement circulaire.

La structure alvéolaire de stimulation de la face plantaire du pied est configurée pour être en contact direct avec le pied, notamment la face plantaire du pied. Une séparation, notamment par le biais d'un revêtement épais supérieur à 3 mm, annihilerait l'effet stimulant de ladite structure alvéolaire.

La semelle orthopédique comprend deux extrémités : une extrémité antérieure et une extrémité postérieure. L'extrémité antérieure correspond à l'extrémité de la semelle située à l'avant des orteils et l'extrémité postérieure correspond à l'extrémité de la semelle située à l'arrière du talon.

La première zone des orteils s'étend de l'extrémité antérieure de la semelle jusqu'à l'avant des têtes métatarsiennes. Elle comprend les phalanges, et peut donc aussi être appelée « zone des phalanges ».

La deuxième zone des têtes métatarsiennes est située au niveau des têtes métatarsiennes.

La troisième zone de barre rétro-capitale s'étend du col des cinq métatarsiens jusqu'à l'interligne de l'articulation de Lisfranc. Elle peut aussi être nommée « troisième zone métatarsienne », comprenant un élément de barre rétro-capitale (BRC). De préférence, la troisième zone de barre rétro-capitale a un relief en aile d'avion dans le plan sagittal avec un maximum de hauteur sur sa partie antérieur et un minimum de hauteur au niveau de l'articulation de Lisfranc. Cette troisième zone de barre rétro-capitale permet de décharger les pressions sur les têtes métatarsiennes, *i.e.* la deuxième zone, qui représente une zone du pied très sensible et/ou fragile, notamment chez les personnes âgées. Cette troisième zone de barre rétro-capitale a donc en premier lieu un rôle de répartition des appuis et de confort au niveau de la plante du pied. Si une première structure alvéolaire de stimulation est présente dans ladite troisième zone, un second rôle de stimulation est alors ajouté.

La zone de stimulation est solidaire, fait partie intégrante de la face plantaire de la semelle. En d'autres termes, il ne s'agit pas d'un insert à ajouter sur la semelle. Avantageusement, cela permet une meilleure solidité de la semelle et prévient tout déplacement possible de la zone de stimulation pendant l'utilisation. Cela permet également une plus grande stimulation car la partie stimulante de la semelle sera plus profonde. Avec une telle zone de stimulation solidaire de la semelle, le pied du porteur va écraser davantage les alvéoles, notamment en profondeur, et offrira une meilleure stimulation de la face plantaire.

La présence d'au moins un trou dans une alvéole permet d'éviter tout effet de succion ou aspiration sur la peau de la face plantaire du pied lors du contact entre les parois de ladite alvéole et la peau. En outre, cela permet également d'accroître la flexibilité de la semelle, notamment car la semelle présente des zones affinées au niveau des trous. Cela permet un plus grand confort pour le porteur et d'éviter des marques ou blessures sur la peau. En effet, la stimulation des mécanorécepteurs doit se faire dans le respect du confort et de la santé du porteur. Les sujets souffrants de troubles de l'équilibre (personnes âgées, souffrant de pathologies neuro-dégénératives ou de neuropathie périphérique) ont déjà la plante des pieds fragilisées et présentent une cicatrisation ralentie, il est donc primordial d'éviter tout risque de blessure (ou de plaie), notamment par succion.

Selon un mode de réalisation, la partie antérieure de la quatrième zone se situe au niveau des os suivants : le naviculaire, le cunéiforme médial, le cunéiforme intermédiaire, le cunéiforme latéral et le cuboïde, et ladite partie postérieure comprend uniquement l'anneau talonnier.

Selon un mode de réalisation, la cinquième zone a une forme ovale, ou une forme de goutte. Cette zone est située au niveau du tarse postérieur, *i.e.* sous le calcanéus, et est orientée vers l'os naviculaire, qui est lui-même compris dans la voûte plantaire.

La semelle orthopédique selon l'invention est une semelle orthopédique proprioceptive, c'est-à-dire un dispositif plantaire fondé sur le principe de proprioception. La proprioception est la perception, consciente ou non, qu'un sujet a de son propre corps dans l'espace. Appliquée aux pieds et à la marche de manière générale, celle-ci est donc la façon dont un sujet perçoit son propre être et sa posture par l'intermédiaire de ses pas et du contact entre le sol et la plante de ses pieds. Les semelles proprioceptives selon l'invention sont ainsi des semelles pourvues d'au moins une zone de stimulation permettant une stimulation optimale et permanente des mécanorécepteurs de la face plantaire du pied, et garantissant, de fait, une autocorrection posturale et une prise en compte naturelle de l'ensemble du corps par le sujet. Autrement dit, la stimulation de la plante du pied créée par la structure alvéolaire de stimulation permet au sujet d'augmenter son afférence plantaire et d'ajuster instinctivement sa position globale. Cela est d'autant plus primordial dans le cas de sujets souffrants de troubles de l'équilibre dont la cause provient de troubles de sensibilité périphérique, comme par exemple : les personnes âgées, les personnes souffrants de pathologies neuro-dégénératives (parkinson, sclérose en plaque et maladie de Charcot Marie Tooth, alzheimer) ou les personnes souffrant de neuropathie périphérique (personnes diabétiques et personnes ayant rencontré un accident comme un AVC par exemple).

La stimulation des mécanorécepteurs par la structure alvéolaire de stimulation est créée par le mouvement des alvéoles lors de la marche ou en position orthostatique. En effet, sous l'effet de la pression du pied, des alvéoles de la structure alvéolaire de stimulation vont s'affaisser et produire ainsi un mouvement transversal à la face plantaire de la semelle, les parois de ces alvéoles vont également se déformer dans le plan de la face plantaire de la semelle et produire ainsi un double mouvement latéral. En effet, le mouvement transversal à la face plantaire de la semelle correspond à un mouvement selon l'axe anatomique longitudinal, désignant ainsi un mouvement vertical, et le double mouvement latéral dans le plan de la face plantaire de la semelle correspond à un mouvement selon l'axe anatomique antéro-postérieur et à un mouvement selon l'axe anatomique sagittal. Ce mouvement des alvéoles dans les trois directions de l'espace permet une stimulation optimale des mécanorécepteurs de la face plantaire du pied. Lorsque la pression du pied cesse sur une zone de la semelle, par exemple pendant un pas ou un repositionnement du pied, les alvéoles retrouvent leur position et forme initiale. Au prochain pas ou repositionnement du pied, d'autres alvéoles ou les mêmes, mais seulement en partie, subiront la pression appliquée par le pied et seront alors déformées dans les 3 directions de l'espace. Le mouvement des alvéoles n'est donc jamais créé au même endroit de la zone de stimulation et évolue à chaque pas, permettant d'éviter la saturation des mécanorécepteurs de la face plantaire du pied, ou une saturation trop rapide des mécanorécepteurs de la face plantaire du pied. Cela a pour avantage d'avoir un effet bénéfique sur la posture et l'équilibre du porteur car une saturation des mécanorécepteurs rendrait inopérant le rééquilibrage inconscient du sujet. Il y a également une dimension de confort car une stimulation perpétuelle à un endroit précis ne serait pas supportable, d'autant plus sur des zones fragiles comme celle des têtes métatarsiennes.

La semelle de l'invention permet donc, par le biais d'au moins une zone de stimulation, de stimuler ces mécanorécepteurs lors de la marche et en position debout statique afin d'améliorer l'information de la position du corps dans l'espace du porteur et lui permettant de déclencher la réponse musculaire adéquat afin de se repositionner pour éviter un déséquilibre pouvant mener à une chute.

En d'autres termes, la structure alvéolaire de stimulation est une surface de stimulation, cette surface étant partielle ou totale sur la semelle, permettant d'augmenter l'afférence podale du porteur.

Les mécanorécepteurs stimulés par la semelle orthopédique selon l'invention ne sont pas des nocicepteurs. En effet, lors de la stimulation des mécanorécepteurs permettant une amélioration de la rétroaction sensorielle plantaire, il est primordial d'éviter toute stimulation nociceptive de la plante des pieds, et en particulier de la voûte plantaire. Le but d'une stimulation nociceptive est de déclencher une réaction du système nerveux central en réaction à un stimulus douloureux qui active les nocicepteurs, autrement dit les récepteurs de la douleur. Il n'est pas question de stimulation nociceptive, i.e. faisant appel aux récepteurs de la douleur, dans la présente invention. Le but de l'invention est de fournir une semelle orthopédique pouvant être portée sur une longue période (par exemple tout au long de la journée) et un tel inconfort rendrait le port de la semelle orthopédique compliqué dû à la douleur. La stimulation des mécanorécepteurs doit donc se faire dans le respect du confort de l'utilisateur, i.e. des sujets souffrants de troubles de l'équilibre dont la cause provient de troubles de sensibilité périphérique, comme par exemple : les personnes âgées, les personnes souffrants de pathologies neuro-dégénératives ou les personnes souffrant de neuropathie périphérique. Ces personnes ont déjà la plante des pieds fragilisées, une stimulation nociceptive n'est donc pas appropriée et devrait être évitée à tout prix. De plus, une stimulation nociceptive peut permettre de contrer le mouvement de l'affaissement du pied en interne, soit la valgisation du pied ou le pied plat : lorsque le pied a tendance à s'affaisser vers l'intérieur, la stimulation nociceptive empêchera le pied de continuer à s'affaisser vers l'intérieur et le repositionnera vers l'extérieur (en varus). Ce n'est pas l'objectif de la présente invention. En effet, la correction de troubles dit statiques n'est pas recherchée ici, l'objectif principal de la présente invention étant l'aide au maintien de l'équilibre postural de la personne (et donc au maintien de l'autonomie, à l'amélioration de la perception des sols, de la position de son corps dans l'espace) peu importe le positionnement de son pied.

Selon un mode de réalisation, la semelle orthopédique a une épaisseur comprise entre 0.5 mm et 10 mm d'épaisseur, préférentiellement entre 0.8 mm et 5 mm d'épaisseur, plus préférentiellement l'épaisseur de la semelle orthopédique est 1.5 mm. Une telle finesse de la semelle orthopédique la rend adaptable à tout type d'éléments chaussants, à savoir tout type de chaussures ou chaussons. L'épaisseur ainsi décrite ne comprend pas la hauteur des alvéoles, de l'anneau talonnier ou du soutien de voûte plantaire. En d'autres mots, il s'agit de la semelle orthopédique « nue ».

Selon une configuration spécifique de ce mode de réalisation, au moins 50% des alvéoles de la première structure alvéolaire de stimulation, *i.e.* la moitié de ces alvéoles, comprennent chacune au moins un trou.

Selon une configuration préférée de ce mode de réalisation, chaque alvéole de la première structure alvéolaire de stimulation, i.e. la totalité des alvéoles, comprend au moins un trou.

Selon une configuration spécifique de ce mode de réalisation, chaque alvéole de la première structure alvéolaire de stimulation comprend une pluralité de trous.

Selon une configuration préférée de ce mode de réalisation, chaque alvéole de la première structure alvéolaire de stimulation comprend un trou.

Selon un mode de réalisation, le diamètre dudit trou est inférieur au diamètre de l'alvéole qui le comprend.

Dans une configuration préférée de ce mode de réalisation, le diamètre dudit trou est inférieur à 6 mm, de préférence compris entre 0.2 mm et 5.9 mm, plus préférentiellement entre 0.5 mm et 5 mm.

Selon un mode de réalisation, l'au moins une zone de stimulation est choisie parmi la première zone, la troisième zone ou la cinquième zone de la semelle.

De préférence, l'au moins une zone de stimulation n'est pas la quatrième zone de la semelle. En d'autres termes, la zone de la voûte plantaire n'est pas stimulée. En particulier, la peau située sous la voûte plantaire est plus fine que sur les autres zones de la face plantaire donc plus fragile et plus sensible. Pour éviter toute douleur provoquée au porteur, il convient donc de ne pas stimuler cette zone particulière. Également, présentant moins de mécanorécepteurs que les autres zones de la face plantaire, la voûte plantaire présente moins d'intérêt pour la stimulation.

Selon une configuration préférée de ce mode de réalisation, l'au moins une zone de stimulation est la première zone. Il y a de nombreux mécanorécepteurs situés au niveau des orteils. De plus, la peau au niveau des orteils est fine et le seuil de perception à la pression est assez bas. Les orteils représentent également une zone importante dans l'équilibre postural, ainsi lors de la marche et du passage du pas (phase dite de propulsion) seuls les orteils restent en contact avec le sol. Une zone de stimulation à cet endroit-ci permet aussi mécaniquement d'augmenter la surface d'appui des orteils qui sont souvent déformés chez les personnes âgées, permettant aux personnes de favoriser leur perception sensitive de l'environnement sur lequel ils sont (matière du sol, position du pied etc...) cela dans le but de favoriser un meilleur équilibre postural.

Selon une autre configuration préférée de ce mode de réalisation, l'au moins une zone de stimulation est la troisième zone. Il y a de nombreux mécanorécepteurs situés au niveau de la BRC. De plus, la peau y est fine et le seuil de perception à la pression y est assez bas.

Selon un mode de réalisation, la semelle comprend au moins deux zones de stimulation choisies parmi la première zone, la troisième zone et/ou la cinquième zone de la semelle.

Selon une configuration spécifique de ce mode de réalisation, les deux zones de stimulation sont la première zone et la troisième zone de la semelle.

Selon une autre configuration spécifique de ce mode de réalisation, les deux zones de stimulation sont la troisième zone et la cinquième zone de la semelle.

Selon une autre configuration spécifique de ce mode de réalisation, les deux zones de stimulation sont la première zone et la cinquième zone de la semelle. Dans ce mode de réalisation, la troisième zone de la semelle ne joue pas de rôle de stimulation et joue pleinement son rôle de décharge de la deuxième zone.

Selon un mode de réalisation, la première zone, la troisième zone et la cinquième zone de la semelle sont des zones de stimulation. Ces trois zones possèdent avantageusement le plus de mécanorécepteurs sur la face plantaire du pied, permettant une stimulation optimale desdits mécanorécepteurs et donc une correction posturale améliorée. Dans ce mode de réalisation, la structure alvéolaire de stimulation est une surface de stimulation partielle sur la semelle, permettant d'augmenter l'afférence podale du porteur.

Selon une configuration préférée de ce mode de réalisation, chaque alvéole de la première structure alvéolaire de stimulation comprend un trou, i.e. chaque alvéole de la première zone, la troisième zone et la cinquième zone de la semelle comprend un trou.

Selon un mode de réalisation, au moins une zone de stimulation comprend en outre des éléments de stimulation répartis dans les alvéoles de la première structure alvéolaire de stimulation. Ces éléments de stimulation ont l'avantage d'accroître la stimulation des mécanorécepteurs, notamment en accentuant l'effet de pression appliquée auxdits mécanorécepteurs. Cet accent porté à l'effet de pression combiné au massage dans les trois directions de l'espace exercé par les alvéoles de la zone de stimulation permet de décupler la stimulation des mécanorécepteurs.

Selon une configuration préférée de ce mode de réalisation, l'au moins une zone de stimulation comprenant en outre des éléments de stimulation répartis dans les alvéoles de la première structure alvéolaire de stimulation est choisie parmi la première zone, la troisième zone et/ou la cinquième zone de la semelle. Ces trois zones possèdent avantageusement le plus de mécanorécepteurs sur la face plantaire du pied, permettant une stimulation optimale desdits mécanorécepteurs et donc une correction posturale améliorée.

Selon un mode de réalisation, les éléments de stimulation sont répartis de manière aléatoire dans les alvéoles de la première structure alvéolaire de stimulation. Une répartition aléatoire des éléments de stimulation dans les alvéoles permet de conserver ledit mouvement des alvéoles dans les 3 directions. En effet, si toutes les alvéoles comprenaient chacune un élément de stimulation, la structure alvéolaire serait beaucoup plus rigide, le mouvement moindre et aussi plus régulier. La stimulation des mécanorécepteurs ne serait alors pas optimale.

Dans une configuration spécifique de ce mode de réalisation, les éléments de stimulation sont répartis de manière à avoir au moins une alvéole vide, de préférence au moins deux alvéoles vides, entre deux alvéoles comprenant chacune un élément de stimulation. Une alvéole vide s'entend ici d'une alvéole ne comprenant pas d'élément de stimulation. Cet espacement des éléments de stimulation permet de prévenir la rigidité de la structure alvéolaire tout en offrant une stimulation augmentée.

Dans une autre configuration spécifique de ce mode de réalisation, le ratio alvéole pleine/alvéole vide est compris entre 1/10 et 1/2, de préférence entre 1/8 et 1/3, plus préférentiellement entre 1/6 et 1/4.

Dans une configuration préférée de ce mode de réalisation, le ratio alvéole pleine/alvéole vide est 1/2, de préférence 1/3, plus préférentiellement 1/5, encore plus préférentiellement 1/6.

Selon un mode de réalisation, les éléments de stimulation ont une forme sphérique, hémisphérique, conique, parallélépipédique, polyédrique, pyramidale, ou cylindrique.

Selon un mode de réalisation, les éléments de stimulation sont des picots.

Selon une configuration spécifique de ce mode de réalisation, une forme conique se réfère à un cône ou un cône avec sommet arrondi, et une forme pyramidale se réfère à une pyramide, une pyramide tronquée ou une pyramide avec sommet arrondi.

Selon une autre configuration spécifique de ce mode de réalisation, une forme polyédrique se réfère à un tétraèdre, un octaèdre, un pentaèdre ou toute autre polyèdre connu.

Selon une configuration préférée de ce mode de réalisation, les éléments de stimulation ont une forme hémisphérique, i.e. les éléments de stimulation sont des demi-billes (ou demi-sphères). De préférence, ces éléments de stimulation sont des demi-sphères pleines. L'avantage des demi sphères est d'augmenter la surface de contact et de stimulation sous le pied et donc d'être moins douloureux qu'un élément à sommet pointu et également de saturer moins vite les mécanorécepteurs.

Selon un mode de réalisation, les éléments de stimulation sont des alvéoles comprises dans les alvéoles de la première structure alvéolaire de stimulation. Ces alvéoles de stimulation étant de diamètre inférieur à celles de la première structure alvéolaire de stimulation. La hauteur de ces alvéoles peut être égale ou inférieure à celle de la première structure alvéolaire de stimulation. Avantageusement, un mouvement supplémentaire et une pression supplémentaire dans les 3 directions de l'espace est créé à l'intérieur même des alvéoles de la première structure alvéolaire de stimulation.

Selon une configuration préférée de ce mode de réalisation, les éléments de stimulation ont un diamètre égal ou inférieur à celui des alvéoles.

Selon une configuration préférée de ce mode de réalisation, les éléments de stimulation ont un diamètre compris entre 0.5 mm et 20 mm, préférentiellement entre 2 mm et 15 mm, plus préférentiellement entre 3 mm et 10 mm.

Selon une configuration préférée de ce mode de réalisation, les éléments de stimulation ont une hauteur égale ou inférieure à celle des parois des alvéoles.

Selon une configuration préférée de ce mode de réalisation, les éléments de stimulation ont une hauteur comprise entre 0.5 mm et 10 mm, préférentiellement entre 1 mm et 5 mm, encore plus préférentiellement entre 1 mm et 3 mm.

Selon une configuration préférée de ce mode de réalisation, les éléments de stimulation sont constitués de silicone, caoutchouc, polychlorure de vinyle (PVC), polyethersulfone (PES), éthylène-acétate de vinyle (EVA), polyuréthane (PU), polyéthylène (PE), latex, ou un mélange de ceux-ci.

Selon un mode de réalisation, la cinquième zone de la semelle comprend en outre des éléments de stimulation répartis dans les alvéoles de la première structure alvéolaire de stimulation, et les première et troisième zones de la semelle comprennent chacune une première structure alvéolaire de stimulation vide. Avantageusement, ces trois zones de la semelle, comprenant un très grand nombre de mécanorécepteurs, ont le rôle de stimulation desdits mécanorécepteurs. La stimulation au niveau de la cinquième zone est accrue par la présence d'éléments de stimulation dans les alvéoles.

Selon un mode de réalisation, la première zone et la cinquième zone de la semelle comprennent en outre des éléments de stimulation répartis dans les alvéoles de leur première structure alvéolaire de stimulation, et la troisième zone de la semelle comprend une première structure alvéolaire de stimulation vide. Avantageusement, ces trois zones de la semelle, comprenant un très grand nombre de mécanorécepteurs, ont le rôle de stimulation desdits mécanorécepteurs. La stimulation au niveau des première et cinquième zones est accrue par la présence d'éléments de stimulation dans les alvéoles tandis que la troisième zone conserve son rôle de décharge de la deuxième zone tout en offrant une stimulation des mécanorécepteurs de la troisième zone. Le but est de conserver une certaine souplesse au niveau de la troisième zone afin d'éviter une stimulation douloureuse sur cette zone sensible et également de favoriser une meilleure stimulation des mecanorécepteurs en optimisant le mouvement de la structure de stimulation.

Selon un mode de réalisation, la troisième zone et la cinquième zone de la semelle comprennent en outre des éléments de stimulation répartis dans les alvéoles de leur première structure alvéolaire de stimulation et la première zone de la semelle comprend une première structure alvéolaire de stimulation vide.

Selon un mode de réalisation, la première zone et la troisième zone de la semelle comprennent en outre des éléments de stimulation répartis dans les alvéoles de leur première structure alvéolaire de stimulation et la cinquième zone de la semelle comprend une première structure alvéolaire de stimulation vide.

Selon un mode de réalisation, les éléments de stimulation répartis dans des zones de stimulation différentes peuvent avoir une forme, et/ou au moins une dimension différente. Les mécanorécepteurs n'étant pas tous localisés à la même profondeur sous la peau, il peut être avantageux, pour atteindre un maximum de ces mécanorécepteurs d'avoir des éléments de stimulation de forme ou de hauteur différentes selon les zones de stimulation. Par exemple, la cinquième zone étant moins sensible à la pression que la première ou la troisième zone, il peut être avantageux d'y placer des éléments de stimulation ayant une forme accentuant plus la pression comme par exemple des demi-sphères ou des cônes.

Selon un mode de réalisation, les éléments de stimulation répartis dans des zones de stimulation différentes sont de forme et dimension identique. Ce mode de réalisation est particulièrement avantageux car les sensations sont plus homogènes pour le porteur de la semelle, permettant un plus grand confort.

Selon un mode de réalisation, au moins une zone de la semelle comprend une seconde structure alvéolaire de stimulation de la face plantaire du pied, ladite seconde structure alvéolaire de stimulation comprenant une pluralité d'alvéoles, chacune délimitée par au moins une paroi s'étendant transversalement à la face plantaire.

Selon un mode de réalisation, l'au moins une zone comprenant ladite seconde structure alvéolaire de stimulation est choisie parmi la deuxième zone ou la quatrième zone de la semelle.

Selon un mode de réalisation, la deuxième zone et la quatrième zone de la semelle comprennent chacune une seconde structure alvéolaire de stimulation.

Selon un mode de réalisation, chaque alvéole de la seconde structure alvéolaire de stimulation comprend un trou. Les trous présents dans la seconde structure alvéolaire de stimulation peuvent avoir un diamètre identique ou différent à celui des trous présents dans la première structure alvéolaire de stimulation.

Selon une configuration préférée de ce mode de réalisation, la première zone, la troisième zone et la cinquième zone de la semelle sont des zones de stimulation comprenant une première structure alvéolaire de stimulation, et la deuxième zone et la quatrième zone de la semelle comprennent chacune une seconde structure alvéolaire de stimulation. Avantageusement, toute la face plantaire du pied est stimulée, avec une stimulation plus douce au niveau des deuxième et quatrième zone. Le mouvement créé par les alvéoles est présent sur toute la face plantaire du pied, sans pour autant induire une sensibilité ou douleur sur les zones sensibles telles que la deuxième et la quatrième zones. Dans ce mode de réalisation, la structure alvéolaire de stimulation est une surface de stimulation totale sur la semelle, permettant d'augmenter l'afférence podale du porteur.

Selon une configuration préférée de ce mode de réalisation, chaque alvéole de la première structure alvéolaire de stimulation comprend un trou, i.e. chaque alvéole de la première zone, la troisième zone et la cinquième zone de la semelle comprend un trou, et les alvéoles de la seconde structure alvéolaire de stimulation (deuxième zone et quatrième zone de la semelle) ne comprennent pas de trou.

Selon une autre configuration préférée de ce mode de réalisation, la première zone et la cinquième zone de la semelle comprennent des éléments de stimulation répartis dans les alvéoles de leur première structure alvéolaire de stimulation, la troisième zone de la semelle comprend une première structure alvéolaire de stimulation vide, et la deuxième zone et la quatrième zone de la semelle comprennent chacune une seconde structure alvéolaire de stimulation.

Selon une autre configuration préférée de ce mode de réalisation, chaque alvéole de la première structure alvéolaire de stimulation comprend un trou ; la première zone et la cinquième zone de la semelle comprennent en outre des éléments de stimulation répartis dans les alvéoles de leur première structure alvéolaire de stimulation ; la troisième zone de la semelle comprend une première structure alvéolaire de stimulation vide ; et la deuxième zone et la quatrième zone de la semelle comprennent chacune une seconde structure alvéolaire de stimulation ne comprenant ni trou ni élément de stimulation.

Selon une autre configuration préférée de ce mode de réalisation, chaque alvéole de la première structure alvéolaire de stimulation et de la seconde structure alvéolaire de stimulation comprend un trou, i.e. chaque alvéole de la première zone, la troisième zone et la cinquième zone de la semelle comprend un trou, et chaque alvéole de la deuxième zone et quatrième zone de la semelle comprend un trou.

Selon un mode de réalisation, la hauteur des parois des alvéoles de la seconde structure alvéolaire de stimulation est plus faible que celle des parois des alvéoles de la première structure alvéolaire de stimulation. Une hauteur plus faible permet de stimuler les mécanorécepteurs sans pour autant induire une douleur, notamment sur des zones particulièrement sensibles comme la deuxième et la quatrième zones. De plus, le fait d'avoir une première structure alvéolaire de hauteur plus importante que la seconde structure alvéolaire va permette de décharger les zones de seconde structure alvéolaire.

Selon un mode de réalisation, la hauteur des parois des alvéoles de la première structure alvéolaire de stimulation est comprise entre 0.5 mm et 10 mm, préférentiellement entre 1 mm et 7.5 mm, plus préférentiellement entre 1 mm et 5 mm.

Selon une configuration préférée de ce mode de réalisation, la hauteur des parois des alvéoles de la première structure alvéolaire de stimulation est 3 mm. Une telle hauteur permet avantageusement de créer un mouvement car les alvéoles sont suffisamment hautes pour s'affaisser sans pour autant être trop hautes ou trop imposantes pour rentrer dans une chaussure ou chausson.

Selon un mode de réalisation, la hauteur des parois des alvéoles de la seconde structure alvéolaire de stimulation est comprise entre 0.5 mm et 3 mm, préférentiellement entre 0.5 mm et 2.9 mm, plus préférentiellement entre 0.8 mm et 1.8 mm.

Selon une configuration préférée de ce mode de réalisation, la hauteur des parois des alvéoles de la seconde structure alvéolaire de stimulation est 1.5 mm, de préférence 1 mm. Une telle hauteur permet avantageusement de créer un mouvement car les alvéoles sont suffisamment hautes pour s'affaisser tout en étant suffisamment basse pour être supportable au niveau de zones sensibles telles que la deuxième et la quatrième zone.

Selon un mode de réalisation, les alvéoles de la première structure alvéolaire de stimulation et/ou la seconde structure alvéolaire de stimulation ont une géométrie hexagonale, pentagonale, circulaire, triangulaire, ou carrée.

Selon une configuration préférée de ce mode de réalisation, les alvéoles de la première structure alvéolaire de stimulation et/ou la seconde structure alvéolaire de stimulation ont une géométrie hexagonale.

Selon un mode de réalisation, la première structure alvéolaire de stimulation et/ou la seconde structure alvéolaire de stimulation a une structure en nid d'abeilles. De façon avantageuse, les nids d'abeilles ont six parois transversales permettant un affaissement dans toutes les directions. De plus, une structure en nid d'abeilles a une grande capacité de compression, *i.e.* est très souple, mais a aussi une grande résistance au cisaillement, *i.e.* est solide. C'est aussi une structure légère car elle comprend de nombreux espaces vides.

Selon une configuration préférée de ce mode de réalisation, la première structure alvéolaire de stimulation et la seconde structure alvéolaire de stimulation a une structure en nid d'abeilles.

Selon un mode de réalisation, les alvéoles de deux zones de stimulation peuvent avoir une géométrie identique, et/ou une hauteur identique ou différente.

Selon un mode de réalisation, les alvéoles de la première structure alvéolaire de stimulation et/ou la seconde structure alvéolaire de stimulation ont une géométrie identique.

Selon un mode de réalisation, le matériau constituant la première structure alvéolaire de stimulation et/ou la seconde structure alvéolaire de stimulation est configuré pour se déformer sous l'action de la pression du pied, par exemple lors de la marche ou de la course, et de retrouver sa forme initiale lorsque ladite pression cesse.

Selon une configuration spécifique de ce mode de réalisation, le matériau constituant la première structure alvéolaire de stimulation et/ou la seconde structure alvéolaire de stimulation est choisi parmi silicone, éthylène-acétate de vinyle (EVA), caoutchouc, polyuréthane (PU), polyéthylène (PE), latex, ou un mélange de ceux-ci.

Selon un mode de réalisation, le diamètre des alvéoles de la première et/ou de la seconde structure alvéolaire de stimulation est compris entre 0.5 mm et 20 mm, préférentiellement entre 3 mm et 15 mm, plus préférentiellement entre 5 mm et 10 mm. Dans le cas d'une alvéole non circulaire, le diamètre est défini comme la distance entre deux parois opposées d'une même alvéole. Une telle gamme de diamètre permet de créer une surface plantaire moins régulière afin d'éviter la saturation des mécanorécepteurs et d'avoir donc un meilleur contrôle postural par une stimulation efficace desdits mécanorécepteurs.

Dans une configuration préférée de ce mode de réalisation, le diamètre des alvéoles de la première et/ou de la seconde structure alvéolaire de stimulation est 6 mm.

Selon un mode de réalisation, la distance centre à centre entre deux alvéoles adjacentes de la première et/ou de la seconde structure alvéolaire de stimulation est comprise entre 0.5 mm et 20 mm, préférentiellement entre 3 mm et 15 mm, plus préférentiellement entre 5 mm et 10 mm.

Dans une configuration préférée de ce mode de réalisation, la distance centre à centre entre deux alvéoles adjacentes de la première et/ou de la seconde structure alvéolaire de stimulation est 6 mm.

Selon un mode de réalisation, l'épaisseur des parois des alvéoles de la première et/ou de la seconde structure alvéolaire de stimulation est compris entre 0.1 mm et 3 mm, préférentiellement entre 0.2 mm et 2 mm, plus préférentiellement entre 0.5 mm et 1.5 mm.

Selon une configuration spécifique de ce mode de réalisation, l'épaisseur des parois des alvéoles de la première structure alvéolaire de stimulation est supérieure à celle des parois des alvéoles de la seconde structure alvéolaire de stimulation.

Selon un mode de réalisation, les alvéoles de la première et/ou de la seconde structure alvéolaire de stimulation sont configurées pour être en contact direct avec la plante du pied.

### Elément chaussant

La présente invention concerne également un élément chaussant comprenant une semelle orthopédique selon l'invention.

La semelle orthopédique et tous ses éléments sont tels que décrits ci-dessus.

Selon un mode de réalisation, l'élément chaussant est une chaussure, un chausson, une botte ou tout autre élément chaussant connu.

Selon un mode de réalisation, la semelle est intégrée à l'élément chaussant.

Selon un mode de réalisation, la semelle selon l'invention est une semelle amovible configurée pour être installée dans ledit élément chaussant.

### Procédé de fabrication

La présente invention concerne également un procédé de fabrication d'une semelle orthopédique selon l'invention.

La semelle orthopédique et tous ses éléments sont tels que décrits ci-dessus.

Selon un mode de réalisation, la semelle selon l'invention est fabriquée par impression 3D en une étape.

Selon un mode de réalisation, la semelle selon l'invention est fabriquée par injection. Dans ce cas, le procédé de fabrication comprend les étapes suivantes :
a) fournir un moule d'une semelle orthopédique selon l'invention
b) injecter le matériau de la semelle orthopédique dans le moule ; et
c) injecter le matériau de l'au moins une première structure alvéolaire de stimulation de la face plantaire du pied dans le moule.

Selon un mode de réalisation, le procédé de fabrication comprend les étapes suivantes :
a) fournir une semelle orthopédique selon l'invention ;
b) fournir au moins une première structure alvéolaire de stimulation de la face plantaire du pied, ladite première structure alvéolaire de stimulation comprenant une pluralité d'alvéoles, chacune délimitée par au moins une paroi s'étendant transversalement à la face plantaire ; et
c) fixer l'au moins une première structure alvéolaire de stimulation sur au moins une zone de la semelle orthopédique.

Selon un mode de réalisation relatif à l'étape a), la semelle orthopédique est de préférence une semelle en EVA.

Selon un mode de réalisation relatif à l'étape a), la semelle orthopédique est obtenue par injection dans un moule.

Selon un mode de réalisation relatif à l'étape b), l'au moins une première structure alvéolaire de stimulation est fabriquée par impression 3D ou injection dans un moule.

Selon un mode de réalisation relatif à l'étape b), l'au moins une première structure alvéolaire de stimulation est fixée sur au moins une zone de la semelle orthopédique par collage, clipsage, assemblage, moulage ou injection.

### BRÈVE DESCRIPTION DES FIGURES

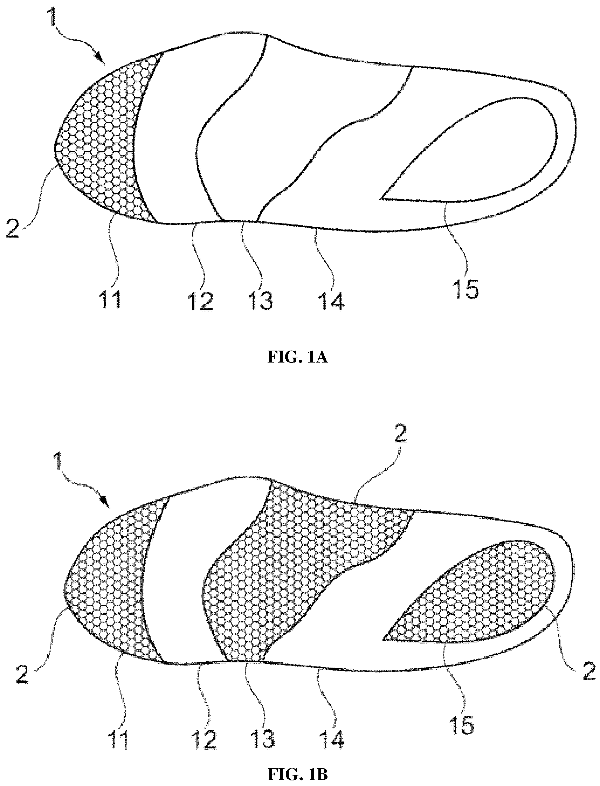

**Figure 1A** est une vue du dessus d'une semelle orthopédique 1 selon un premier mode de réalisation de l'invention, dans laquelle la première zone de la semelle 11 est une zone de stimulation comprenant une première structure alvéolaire de stimulation de la face plantaire du pied 2.
**Figure 1B** est une vue du dessus d'une semelle orthopédique 1 selon un deuxième mode de réalisation de l'invention, dans laquelle la première zone 11, la troisième zone 13 et la cinquième zone 15 de la semelle sont des zones de stimulation comprenant une première structure alvéolaire de stimulation de la face plantaire du pied 2.

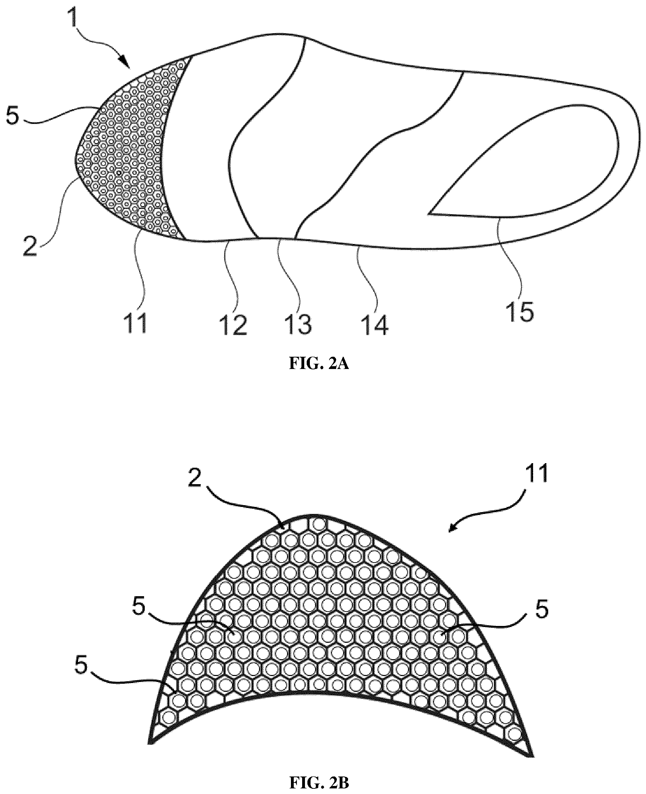

**Figure 2A** est une vue du dessus d'une semelle orthopédique 1 selon un troisième mode de réalisation de l'invention, dans laquelle la première zone de la semelle 11 est une zone de stimulation comprenant une première structure alvéolaire de stimulation de la face plantaire du pied 2, chaque alvéole de ladite première structure alvéolaire de stimulation 2 comprenant un trou 5.
**Figure 2B** est un agrandissement de la première zone de la semelle 11 selon la figure 2A.

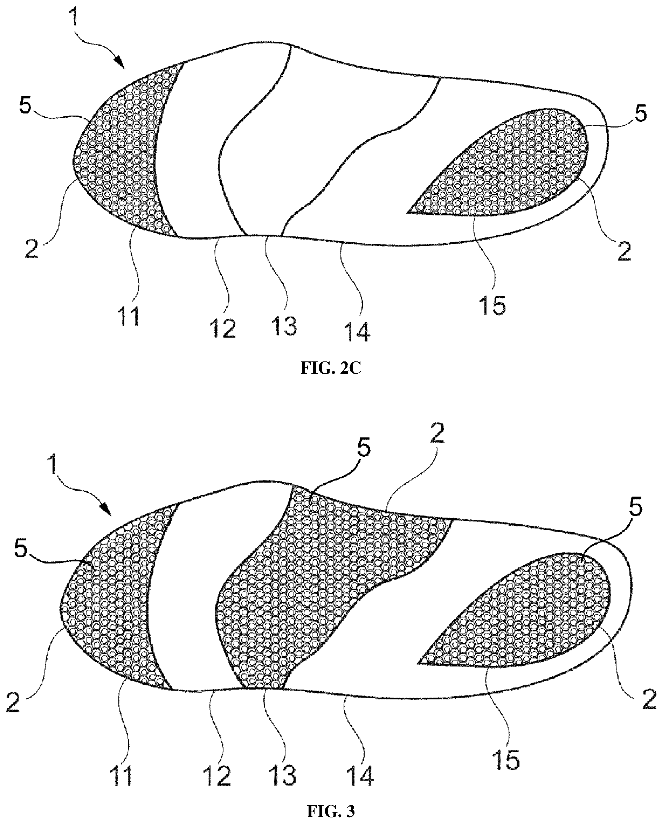

**Figure 2C** est une vue du dessus d'une semelle orthopédique 1 selon un quatrième mode de réalisation de l'invention, dans laquelle la première zone 11 et la cinquième zone 15 de la semelle sont des zones de stimulation comprenant une première structure alvéolaire de stimulation de la face plantaire du pied 2, chaque alvéole de ladite première structure alvéolaire de stimulation 2 comprenant un trou 5.
**Figure 3** est une vue du dessus d'une semelle orthopédique 1 selon un cinquième mode de réalisation de l'invention, dans laquelle la première zone 11, la troisième zone 13 et la cinquième zone 15 de la semelle sont des zones de stimulation comprenant une première structure alvéolaire de stimulation de la face plantaire du pied 2, chaque alvéole de ladite première structure alvéolaire de stimulation 2 comprenant un trou 5.

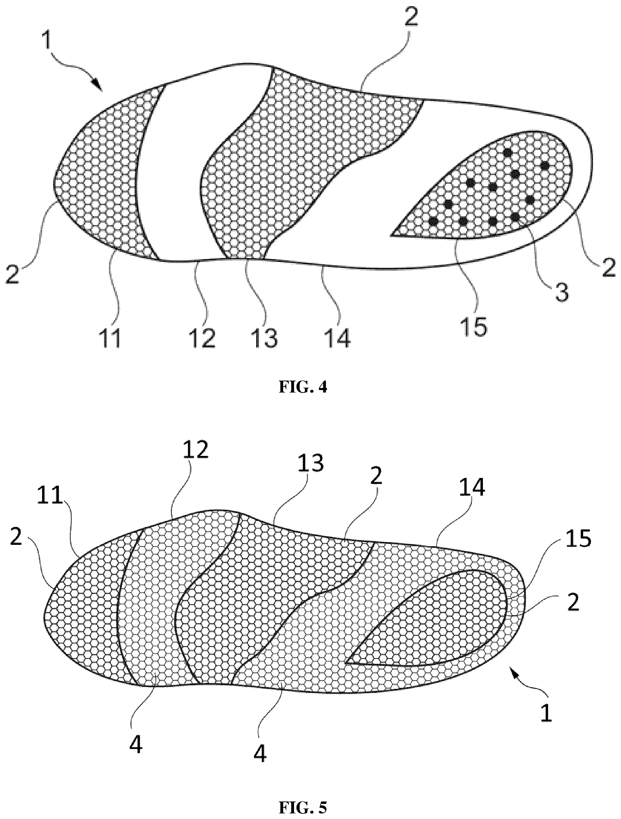

**Figure 4** est une vue du dessus d'une semelle orthopédique 1 selon un sixième mode de réalisation de l'invention, dans laquelle la première zone 11, la troisième zone 13 et la cinquième zone 15 de la semelle sont des zones de stimulation comprenant une première structure alvéolaire de stimulation de la face plantaire du pied 2, et la cinquième zone 15 comprend en outre des éléments de stimulation 3.
**Figure 5** est une vue du dessus d'une semelle orthopédique 1 selon un septième mode de réalisation de l'invention, dans laquelle la première zone 11, la troisième zone 13 et la cinquième zone 15 de la semelle comprennent chacune une première structure alvéolaire de stimulation 2, et la deuxième zone 12 et la quatrième zone 14 de la semelle comprennent chacune une seconde structure alvéolaire de stimulation 4.

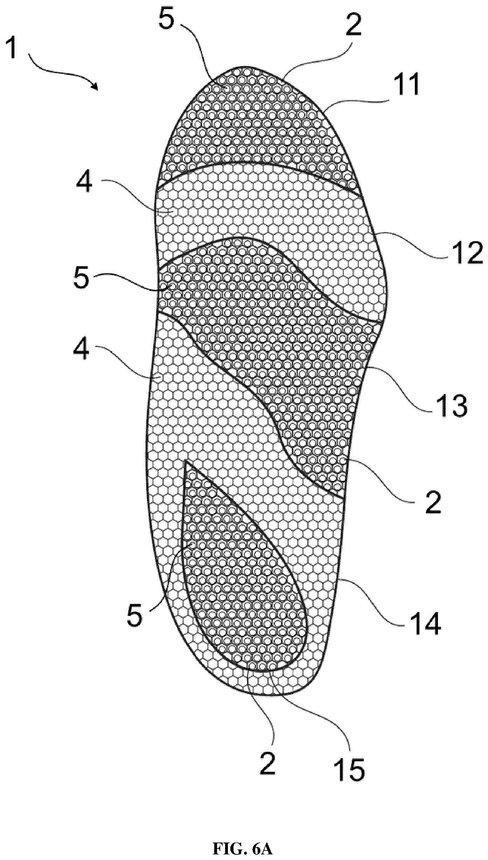

**Figure 6A** est une vue du dessus d'une semelle orthopédique 1 selon un huitième mode de réalisation de l'invention, dans laquelle la première zone 11, la troisième zone 13 et la cinquième zone 15 de la semelle comprennent chacune une première structure alvéolaire de stimulation 2, et la deuxième zone 12 et la quatrième zone 14 de la semelle comprennent chacune une seconde structure alvéolaire de stimulation 4, chaque alvéole de ladite première structure alvéolaire de stimulation 2 comprenant un trou 5.

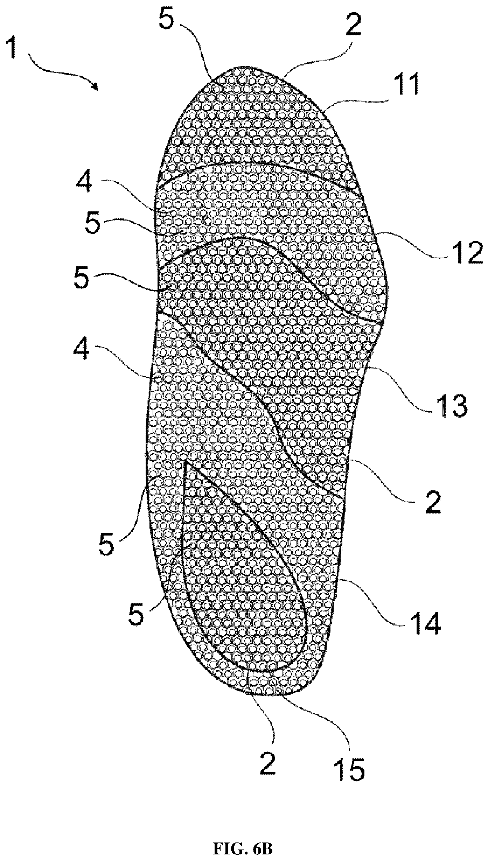

**Figure 6B** est une vue du dessus d'une semelle orthopédique 1 selon un huitième mode de réalisation de l'invention, dans laquelle la première zone 11, la troisième zone 13 et la cinquième zone 15 de la semelle comprennent chacune une première structure alvéolaire de stimulation 2, et la deuxième zone 12 et la quatrième zone 14 de la semelle comprennent chacune une seconde structure alvéolaire de stimulation 4, chaque alvéole de ladite première structure alvéolaire de stimulation 2 et de ladite seconde structure alvéolaire de stimulation 4 comprenant un trou 5.

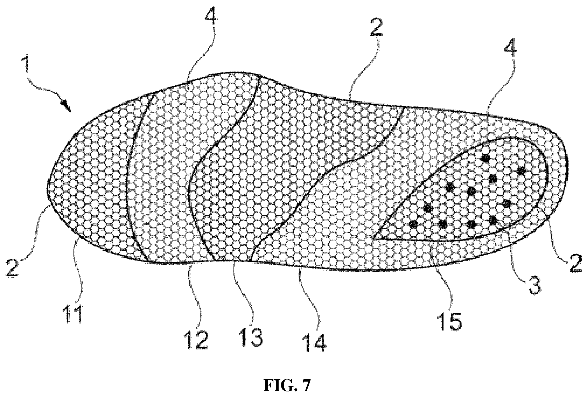

**Figure 7** est une vue du dessus d'une semelle orthopédique 1 selon un neuvième mode de réalisation de l'invention, dans laquelle la première zone 11, la troisième zone 13 et la cinquième zone 15 de la semelle comprennent chacune une première structure alvéolaire de stimulation 2, la cinquième zone 15 comprenant en outre des éléments de stimulation 3, et la deuxième zone 12 et la quatrième zone 14 de la semelle comprennent chacune une seconde structure alvéolaire de stimulation 4.

### MODES DE RÉALISATION ILLUSTRATIFS DE L'INVENTION

Comme illustré sur la figure 1A, la semelle orthopédique 1 selon un premier mode de réalisation de l'invention comprend une face plantaire et une face opposée, ladite face plantaire comprenant consécutivement cinq zones (11, 12, 13, 14, 15) d'une extrémité à l'autre de la semelle 1 :
- une première zone des orteils 11 ;
- une deuxième zone des têtes métatarsiennes 12 ;
- une troisième zone de barre rétro-capitale 13 ;
- une quatrième zone du tarse 14 comprenant une partie antérieure et une partie postérieure, ladite partie antérieure comprenant le tarse antérieur et ladite partie postérieure comprenant un anneau talonnier ;
- une cinquième zone talonnière postéro-interne 15 délimitée par la partie antérieure et la partie postérieure de la quatrième zone 14 ;
dans laquelle la première zone de la semelle 11 est une zone de stimulation comprenant une première structure alvéolaire de stimulation de la face plantaire du pied 2, ladite première structure alvéolaire de stimulation 2 comprenant une pluralité d'alvéoles, chacune délimitée par au moins une paroi s'étendant transversalement à la face plantaire.

Ce mode de réalisation est particulièrement avantageux en ce qu'il permet une stimulation des mécanorécepteurs présents à l'avant du pied, au niveau de la première zone 11 de la semelle par le biais du mouvement dans les trois directions des parois des alvéoles de première structure alvéolaire de stimulation de la face plantaire du pied 2 lorsque ces dernières sont soumises à la pression du pied.

Comme illustré sur la figure 1B, la semelle orthopédique 1 selon un troisième mode de réalisation de l'invention comprend une face plantaire et une face opposée, ladite face plantaire comprenant consécutivement cinq zones (11, 12, 13, 14, 15) d'une extrémité à l'autre de la semelle 1 :
- une première zone des orteils 11 ;
- une deuxième zone des têtes métatarsiennes 12 ;
- une troisième zone de barre rétro-capitale 13 ;
- une quatrième zone du tarse 14 comprenant une partie antérieure et une partie postérieure, ladite partie antérieure comprenant le tarse antérieur et ladite partie postérieure comprenant un anneau talonnier ;
- une cinquième zone talonnière postéro-interne 15 délimitée par la partie antérieure et la partie postérieure de la quatrième zone 14 ;
dans laquelle la première zone 11, la troisième zone 13 et la cinquième zone 15 de la semelle sont des zones de stimulation comprenant une première structure alvéolaire de stimulation de la face plantaire du pied 2, ladite première structure alvéolaire de stimulation 2 comprenant une pluralité d'alvéoles, chacune délimitée par au moins une paroi s'étendant transversalement à la face plantaire.

Ce mode de réalisation est particulièrement avantageux en ce qu'il permet une stimulation des mécanorécepteurs présents dans les première, troisième et cinquième zones (11, 13, 15) par le biais du mouvement dans les trois directions de l'espace des parois des alvéoles de première structure alvéolaire de stimulation de la face plantaire du pied 2 lorsque ces dernières sont soumises à la pression du pied. Ces trois zones (11, 13, 15) présentent un très grand nombre de mécanorécepteurs et représentent des points d'appuis importants au niveau du pied en statique et en dynamique. Cela permet d'avoir une stimulation optimale des mécanorécepteurs.

Comme illustré sur les figure 2A et 2B, la semelle orthopédique 1 selon un premier mode de réalisation de l'invention comprend une face plantaire et une face opposée, ladite face plantaire comprenant consécutivement cinq zones (11, 12, 13, 14, 15) d'une extrémité à l'autre de la semelle 1 :
- une première zone des orteils 11 ;
- une deuxième zone des têtes métatarsiennes 12 ;
- une troisième zone de barre rétro-capitale 13 ;
- une quatrième zone du tarse 14 comprenant une partie antérieure et une partie postérieure, ladite partie antérieure comprenant le tarse antérieur et ladite partie postérieure comprenant un anneau talonnier ;
- une cinquième zone talonnière postéro-interne 15 délimitée par la partie antérieure et la partie postérieure de la quatrième zone 14 ;
dans laquelle la première zone de la semelle 11 est une zone de stimulation comprenant une première structure alvéolaire de stimulation de la face plantaire du pied 2, ladite première structure alvéolaire de stimulation 2 comprenant une pluralité d'alvéoles, chacune délimitée par au moins une paroi s'étendant transversalement à la face plantaire, chaque alvéole de ladite première structure alvéolaire de stimulation 2 comprenant un trou 5.

Ce mode de réalisation est particulièrement avantageux en ce qu'il permet une stimulation des mécanorécepteurs présents à l'avant du pied, au niveau de la première zone 11 de la semelle par le biais du mouvement dans les trois directions des parois des alvéoles de première structure alvéolaire de stimulation de la face plantaire du pied 2 lorsque ces dernières sont soumises à la pression du pied. La présence de trous 5 dans les alvéoles permet plus de confort et d'éviter un effet de succion ou aspiration de la peau du porteur par le contact des alvéoles avec la peau.

Comme illustré sur la figure 2C, la semelle orthopédique 1 selon un deuxième mode de réalisation de l'invention comprend une face plantaire et une face opposée, ladite face plantaire comprenant consécutivement cinq zones (11, 12, 13, 14, 15) d'une extrémité à l'autre de la semelle 1 :
- une première zone des orteils 11 ;
- une deuxième zone des têtes métatarsiennes 12 ;
- une troisième zone de barre rétro-capitale 13 ;
- une quatrième zone du tarse 14 comprenant une partie antérieure et une partie postérieure, ladite partie antérieure comprenant le tarse antérieur et ladite partie postérieure comprenant un anneau talonnier ;
- une cinquième zone talonnière postéro-interne 15 délimitée par la partie antérieure et la partie postérieure de la quatrième zone 14 ;
dans laquelle la première zone 11 et la cinquième zone 15 de la semelle sont des zones de stimulation comprenant une première structure alvéolaire de stimulation de la face plantaire du pied 2, ladite première structure alvéolaire de stimulation 2 comprenant une pluralité d'alvéoles, chacune délimitée par au moins une paroi s'étendant transversalement à la face plantaire, chaque alvéole de ladite première structure alvéolaire de stimulation 2 comprenant un trou 5.

Ce mode de réalisation est particulièrement avantageux en ce qu'il permet une stimulation des mécanorécepteurs présents à l'avant du pied et au niveau du talon par le biais du mouvement dans les trois directions de l'espace des parois des alvéoles de première structure alvéolaire de stimulation de la face plantaire du pied 2 lorsque ces dernières sont soumises à la pression du pied. La présence de trous 5 dans les alvéoles permet plus de confort et d'éviter un effet de succion ou aspiration de la peau du porteur par le contact des alvéoles avec la peau.

Comme illustré sur la figure 3, la semelle orthopédique 1 selon un troisième mode de réalisation de l'invention comprend une face plantaire et une face opposée, ladite face plantaire comprenant consécutivement cinq zones (11, 12, 13, 14, 15) d'une extrémité à l'autre de la semelle 1 :
- une première zone des orteils 11 ;
- une deuxième zone des têtes métatarsiennes 12 ;
- une troisième zone de barre rétro-capitale 13 ;
- une quatrième zone du tarse 14 comprenant une partie antérieure et une partie postérieure, ladite partie antérieure comprenant le tarse antérieur et ladite partie postérieure comprenant un anneau talonnier ;
- une cinquième zone talonnière postéro-interne 15 délimitée par la partie antérieure et la partie postérieure de la quatrième zone 14 ;
dans laquelle la première zone 11, la troisième zone 13 et la cinquième zone 15 de la semelle sont des zones de stimulation comprenant une première structure alvéolaire de stimulation de la face plantaire du pied 2, ladite première structure alvéolaire de stimulation 2 comprenant une pluralité d'alvéoles, chacune délimitée par au moins une paroi s'étendant transversalement à la face plantaire, chaque alvéole de ladite première structure alvéolaire de stimulation 2 comprenant un trou 5.

Ce mode de réalisation est particulièrement avantageux en ce qu'il permet une stimulation des mécanorécepteurs présents dans les première, troisième et cinquième zones (11, 13, 15) par le biais du mouvement dans les trois directions de l'espace des parois des alvéoles de première structure alvéolaire de stimulation de la face plantaire du pied 2 lorsque ces dernières sont soumises à la pression du pied. Ces trois zones (11, 13, 15) présentent un très grand nombre de mécanorécepteurs et représentent des points d'appuis importants au niveau du pied en statique et en dynamique. Cela permet d'avoir une stimulation optimale des mécanorécepteurs. La présence de trous 5 dans les alvéoles permet en outre plus de confort et d'éviter un effet de succion ou aspiration de la peau du porteur par le contact des alvéoles avec la peau.

Comme illustré sur la figure 4, la semelle orthopédique 1 selon un quatrième mode de réalisation de l'invention comprend une face plantaire et une face opposée, ladite face plantaire comprenant consécutivement cinq zones (11, 12, 13, 14, 15) d'une extrémité à l'autre de la semelle 1 :
- une première zone des orteils 11 ;
- une deuxième zone des têtes métatarsiennes 12 ;
- une troisième zone de barre rétro-capitale 13 ;
- une quatrième zone du tarse 14 comprenant une partie antérieure et une partie postérieure, ladite partie antérieure comprenant le tarse antérieur et ladite partie postérieure comprenant un anneau talonnier ;
- une cinquième zone talonnière postéro-interne 15 délimitée par la partie antérieure et la partie postérieure de la quatrième zone 14 ;
dans laquelle la première zone 11, la troisième zone 13 et la cinquième zone 15 de la semelle sont des zones de stimulation comprenant une première structure alvéolaire de stimulation de la face plantaire du pied 2, et la cinquième zone 15 comprend en outre des éléments de stimulation 3.

Ce mode de réalisation est particulièrement avantageux en ce que ces trois zones (11, 13, 15) de la semelle, comprenant un très grand nombre de mécanorécepteurs, ont le rôle de stimulation desdits mécanorécepteurs. La stimulation au niveau de la cinquième zone 15 est accrue par la présence d'éléments de stimulation 3 dans les alvéoles tandis que la troisième zone 13 conserve son rôle de décharge de la deuxième zone 12 tout en offrant une stimulation des mécanorécepteurs de la troisième zone 13. La troisième zone 13 et la première zone 11 sont également des zones sensibles d'un point de vue cutané, c'est pour cela que ces zones ne comprennent, de préférence, pas d'éléments de stimulation 3, afin d'éviter toute blessure ou douleur. Par ailleurs le fait de ne pas y inclure d'éléments de stimulation permet de garder une structure de semelle 1 très souple et flexible.

Comme illustré sur la figure 5, la semelle orthopédique 1 selon un cinquième mode de réalisation de l'invention comprend une face plantaire et une face opposée, ladite face plantaire comprenant consécutivement cinq zones (11, 12, 13, 14, 15) d'une extrémité à l'autre de la semelle 1 :
- une première zone des orteils 11 ;
- une deuxième zone des têtes métatarsiennes 12 ;
- une troisième zone de barre rétro-capitale 13 ;
- une quatrième zone du tarse 14 comprenant une partie antérieure et une partie postérieure, ladite partie antérieure comprenant le tarse antérieur et ladite partie postérieure comprenant un anneau talonnier ;
- une cinquième zone talonnière postéro-interne 15 délimitée par la partie antérieure et la partie postérieure de la quatrième zone 14 ;
dans laquelle la première zone 11, la troisième zone 13 et la cinquième zone 15 de la semelle comprennent chacune une première structure alvéolaire de stimulation 2, et la deuxième zone 12 et la quatrième zone 14 de la semelle comprennent chacune une seconde structure alvéolaire de stimulation 4.

La hauteur et l'épaisseur des parois des alvéoles de la seconde structure alvéolaire de stimulation 4 sont plus faibles que celles des parois des alvéoles de la première structure alvéolaire de stimulation 2.

Ce mode réalisation est particulièrement avantageux en ce que toute la face plantaire du pied est stimulée, avec une stimulation plus douce au niveau des deuxième et quatrième zone.

Comme illustré sur la figure 6A, la semelle orthopédique 1 selon un cinquième mode de réalisation de l'invention comprend une face plantaire et une face opposée, ladite face plantaire comprenant consécutivement cinq zones (11, 12, 13, 14, 15) d'une extrémité à l'autre de la semelle 1 :
- une première zone des orteils 11 ;
- une deuxième zone des têtes métatarsiennes 12 ;
- une troisième zone de barre rétro-capitale 13 ;
- une quatrième zone du tarse 14 comprenant une partie antérieure et une partie postérieure, ladite partie antérieure comprenant le tarse antérieur et ladite partie postérieure comprenant un anneau talonnier ;
- une cinquième zone talonnière postéro-interne 15 délimitée par la partie antérieure et la partie postérieure de la quatrième zone 14 ;
dans laquelle la première zone 11, la troisième zone 13 et la cinquième zone 15 de la semelle comprennent chacune une première structure alvéolaire de stimulation 2, et la deuxième zone 12 et la quatrième zone 14 de la semelle comprennent chacune une seconde structure alvéolaire de stimulation 4.

Chaque alvéole de ladite première structure alvéolaire de stimulation 2 comprend un trou 5. Les alvéoles de la seconde structure alvéolaire de stimulation 4 ne comprennent pas de trou 5 car leur faible hauteur prévient tout effet de succion ou aspiration sur la peau.

La hauteur et l'épaisseur des parois des alvéoles de la seconde structure alvéolaire de stimulation 4 sont plus faibles que celles des parois des alvéoles de la première structure alvéolaire de stimulation 2.

Comme illustré sur la figure 6B, la semelle orthopédique 1 selon un cinquième mode de réalisation de l'invention comprend une face plantaire et une face opposée, ladite face plantaire comprenant consécutivement cinq zones (11, 12, 13, 14, 15) d'une extrémité à l'autre de la semelle 1 :
- une première zone des orteils 11 ;
- une deuxième zone des têtes métatarsiennes 12 ;
- une troisième zone de barre rétro-capitale 13 ;
- une quatrième zone du tarse 14 comprenant une partie antérieure et une partie postérieure, ladite partie antérieure comprenant le tarse antérieur et ladite partie postérieure comprenant un anneau talonnier ;
- une cinquième zone talonnière postéro-interne 15 délimitée par la partie antérieure et la partie postérieure de la quatrième zone 14 ;
dans laquelle la première zone 11, la troisième zone 13 et la cinquième zone 15 de la semelle comprennent chacune une première structure alvéolaire de stimulation 2, et la deuxième zone 12 et la quatrième zone 14 de la semelle comprennent chacune une seconde structure alvéolaire de stimulation 4.

Chaque alvéole de ladite première structure alvéolaire de stimulation 2 et de ladite seconde structure alvéolaire de stimulation 4 comprend un trou 5.

La hauteur et l'épaisseur des parois des alvéoles de la seconde structure alvéolaire de stimulation 4 sont plus faibles que celles des parois des alvéoles de la première structure alvéolaire de stimulation 2.

Ces deux modes de réalisation (correspondant aux figures 6A et 6B) sont particulièrement avantageux en ce que toute la face plantaire du pied est stimulée, avec une stimulation plus douce au niveau des deuxième et quatrième zone. La présence de trous 5 dans les alvéoles permet en outre plus de confort et d'éviter un effet de succion ou aspiration de la peau du porteur par le contact des alvéoles avec la peau.

Comme illustré sur la figure 7, la semelle orthopédique 1 selon un sixième mode de réalisation de l'invention comprend une face plantaire et une face opposée, ladite face plantaire comprenant consécutivement cinq zones (11, 12, 13, 14, 15) d'une extrémité à l'autre de la semelle 1 :
- une première zone des orteils 11 ;
- une deuxième zone des têtes métatarsiennes 12 ;
- une troisième zone de barre rétro-capitale 13 ;
- une quatrième zone du tarse 14 comprenant une partie antérieure et une partie postérieure, ladite partie antérieure comprenant le tarse antérieur et ladite partie postérieure comprenant un anneau talonnier ;
- une cinquième zone talonnière postéro-interne 15 délimitée par la partie antérieure et la partie postérieure de la quatrième zone 14 ;
dans laquelle la première zone 11, la troisième zone 13 et la cinquième zone 15 de la semelle comprennent chacune une première structure alvéolaire de stimulation 2, la cinquième zone 15 comprenant en outre des éléments de stimulation 3, et la deuxième zone 12 et la quatrième zone 14 de la semelle comprennent chacune une seconde structure alvéolaire de stimulation 4.

Ce mode de réalisation est particulièrement avantageux en ce que ces trois zones (11, 13, 15) de la semelle, comprenant un très grand nombre de mécanorécepteurs, ont le rôle de stimulation desdits mécanorécepteurs. La stimulation au niveau de la cinquième zone 15 est accrue par la présence d'éléments de stimulation 3 dans les alvéoles tandis que la troisième zone 13 conserve son rôle de décharge de la deuxième zone 12 tout en offrant une stimulation des mécanorécepteurs de la troisième zone 13. Les deux zones restantes (12, 14) sont elles aussi stimulées de manière plus douce, garantissant ainsi une stimulation de la face plantaire du pied dans son intégralité.

### EXEMPLES

La présente invention se comprendra mieux à la lecture des exemples suivants qui illustrent non-limitativement l'invention.

### Exemple 1 : Semelle comprenant des éléments de stimulation

### Matériel et Méthodes

Dix sujets atteints de la maladie de Parkinson ont été équipés d'une paire de semelle selon l'invention, notamment dans laquelle la première zone, la troisième zone et la cinquième zone sont des zones de stimulation comprenant une première structure alvéolaire de stimulation de la face plantaire du pied, et la cinquième zone comprend en outre des éléments de stimulation (voir figure 4).

La semelle est une semelle en EVA.

La structure alvéolaire de stimulation a une structure en nid d'abeilles en caoutchouc, la hauteur des parois des alvéoles est de 3 mm et le diamètre des alvéoles est de 6 mm. Les éléments de stimulation sont des demi-sphères en silicone de 5.8 mm de diamètre et de 3 mm de hauteur.

### Résultats

Les sujets ont porté les semelles de l'invention pendant 1 heure. Il a été observé moins de déséquilibres à la marche et aucune chute. Les sujets ont témoigné d'une meilleure aisance posturale, d'un sentiment de sécurité à la marche et d'un grand confort.

### Exemple 2 : Semelle comprenant deux structures alvéolaires de stimulation

### Matériel et Méthodes

Dix sujets atteints de la maladie de Parkinson ont été équipés d'une paire de semelle selon la figure 6A, notamment dans laquelle :
- la première zone, la troisième zone et la cinquième zone sont des zones de stimulation comprenant une première structure alvéolaire de stimulation de la face plantaire du pied ;
- la deuxième zone et la quatrième zone de la semelle sont des zones de stimulation comprenant chacune une seconde structure alvéolaire de stimulation ;
- chaque alvéole de ladite première structure alvéolaire de stimulation comprennent un trou ;
- les alvéoles de la seconde structure alvéolaire de stimulation ne comprennent pas de trou.

La semelle est une semelle en EVA.

La structure alvéolaire de stimulation a une structure en nid d'abeilles en caoutchouc, la hauteur des parois des alvéoles est de 3 mm et le diamètre des alvéoles est de 6 mm. Les éléments de stimulation sont des demi-sphères en silicone de 5.8 mm de diamètre et de 3 mm de hauteur.

### Résultats

Les sujets ont porté les semelles de l'invention pendant 1 heure. Il a été observé une augmentation de la longueur du pas à la marche, une augmentation de la vitesse de marche, une sensation de confort, une amélioration de l'équilibre postural et une augmentation de la surface d'appui au sol.

Les sujets ont témoigné d'un plus grand confort dû à la présence de trous dans les alvéoles de la première structure alvéolaire de stimulation et aucune marque de succion ou aspiration sur la peau plantaire n'a été observée.

### REFERENCES

1 - Semelle orthopédique
11 - première zone de la semelle orthopédique
12 - deuxième zone de la semelle orthopédique
13 - troisième zone de la semelle orthopédique
14 - quatrième zone de la semelle orthopédique
15 - cinquième zone de la semelle orthopédique
2 - première structure alvéolaire de stimulation de la face plantaire du pied
3 - élément de stimulation
4 - seconde structure alvéolaire de stimulation de la face plantaire du pied
5 - trou

## Revendications

1. Semelle orthopédique (1) comprenant une face plantaire et une face opposée, ladite face plantaire comprenant consécutivement cinq zones d'une extrémité à l'autre de la semelle (1) :
- une première zone des orteils (11) ;
- une deuxième zone des têtes métatarsiennes (12) ;
- une troisième zone de barre rétro-capitale (13) ;
- une quatrième zone du tarse (14) comprenant une partie antérieure et une partie postérieure, ladite partie antérieure comprenant le tarse antérieur et ladite partie postérieure comprenant un anneau talonnier ;
- une cinquième zone talonnière postéro-interne (15) délimitée par la partie antérieure et la partie postérieure de la quatrième zone (14) ;
dans laquelle au moins une de ces zones est une zone de stimulation comprenant une première structure alvéolaire de stimulation de la face plantaire du pied (2), ladite première structure alvéolaire de stimulation (2) comprenant une pluralité d'alvéoles, chacune délimitée par au moins une paroi s'étendant transversalement à la face plantaire ; et **caractérisé en ce que**
dans laquelle au moins une alvéole de la première structure alvéolaire de stimulation (2) comprend au moins un trou (5).

2. La semelle (1) selon la revendication 1, dans laquelle au moins une zone de stimulation est choisie parmi la première zone (11), la troisième zone (13) ou la cinquième zone (15) de la semelle (1).

3. La semelle (1) selon la revendication **1** ou la revendication **2,** dans laquelle la semelle (1) comprend au moins deux zones de stimulation choisies parmi la première zone (11), la troisième zone (13) et/ou la cinquième zone (15) de la semelle (1).

4. La semelle (1) selon l'une quelconque des revendications **1** à **3,** dans laquelle la première zone (11), la troisième zone (13) et la cinquième zone (15) de la semelle (1) sont des zones de stimulation.

5. La semelle (1) selon l'une quelconque des revendications **1** à **4,** dans laquelle au moins une zone de stimulation comprend en outre des éléments de stimulation (3) répartis dans les alvéoles de la première structure alvéolaire de stimulation (2).

6. La semelle (1) selon la revendication **5,** dans laquelle les éléments de stimulation (3) ont une forme sphérique, hémisphérique, conique, parallélépipédique, polyédrique, pyramidale, ou cylindrique.

7. La semelle (1) selon la revendication **5** ou la revendication **6,** dans laquelle la première zone (11) et la cinquième zone (15) de la semelle (1) comprennent en outre des éléments de stimulation (3) répartis dans les alvéoles de leur première structure alvéolaire de stimulation (2) et dans laquelle la troisième zone (13) de la semelle (1) comprend une première structure alvéolaire de stimulation (2) vide.

8. La semelle (1) selon l'une quelconque des revendications **1** à **7,** dans laquelle au moins une zone de la semelle (1) comprend une seconde structure alvéolaire de stimulation de la face plantaire du pied (4), ladite seconde structure alvéolaire de stimulation (4) comprenant une pluralité d'alvéoles, chacune délimitée par au moins une paroi s'étendant transversalement à la face plantaire.

9. La semelle (1) selon la revendication **8,** dans laquelle la au moins une zone comprenant ladite seconde structure alvéolaire de stimulation (4) est choisie parmi la deuxième zone (12) ou la quatrième zone (14) de la semelle (1).

10. La semelle (1) selon l'une quelconque des revendications **8** ou **9,** dans laquelle la deuxième zone (12) et la quatrième zone (14) de la semelle (1) comprennent chacune une seconde structure alvéolaire de stimulation (4).

11. La semelle (1) selon l'une quelconque des revendications **8** à **10,** dans laquelle la hauteur des parois des alvéoles de la seconde structure alvéolaire de stimulation (4) est plus faible que celle des parois des alvéoles de la première structure alvéolaire de stimulation (2).

12. La semelle (1) selon l'une quelconque des revendications **1** à **11,** dans laquelle la première structure alvéolaire de stimulation (2) et/ou la seconde structure alvéolaire de stimulation (4) a une structure en nid d'abeilles.

13. Elément chaussant comprenant une semelle orthopédique (1) selon l'une quelconque des revendications **1** à **12.**

14. Procédé de fabrication d'une semelle orthopédique (1) selon l'une quelconque des revendications **1** à **12.**

## Patentansprüche

1. Orthopädische Sohle (1), die eine Fußfläche und eine gegenüberliegende Seite umfasst, wobei die Fußfläche nacheinander fünf Bereiche von einem Ende zum anderen der Sohle (1) umfasst:
- einen ersten Bereich der Zehen (11);
- einen zweiten Bereich der Mittelfußköpfe (12);
- einen dritten Bereich der retrokapitalen Pelotte (13);
- einen vierten Bereich des Tarsus (14), der einen vorderen Teil und einen hinteren Teil umfasst, wobei der vordere Teil den vorderen Tarsus umfasst und der hintere Teil einen Fersenring umfasst;
- einen fünften posterioren inneren Fersenbereich (15), der durch den vorderen Teil und den hinteren Teil des vierten Bereichs (14) begrenzt ist;
wobei mindestens einer dieser Bereiche ein Stimulationsbereich ist, der eine erste Stimulationswabenstruktur der Fußfläche des Fußes (2) umfasst, wobei die erste Stimulationswabenstruktur (2) eine Vielzahl von Waben umfasst, die jeweils von mindestens einer Wand begrenzt sind, die sich quer zur Fußfläche erstreckt; und **dadurch gekennzeichnet ist, dass**
die mindestens eine Wabe der ersten Stimulationswabenstruktur (2) mindestens ein Loch (5) umfasst.

2. Sohle (1) nach Anspruch **1,** wobei mindestens ein Stimulationsbereich aus dem ersten Bereich (11), dem dritten Bereich (13) oder dem fünften Bereich (15) der Sohle (1) ausgewählt ist.

3. Sohle (1) nach Anspruch **1** oder Anspruch **2,** wobei die Sohle (1) mindestens zwei Stimulationsbereiche umfasst, die aus dem ersten Bereich (11), dem dritten Bereich (13) und/oder dem fünften Bereich (15) der Sohle (1) ausgewählt sind.

4. Sohle (1) nach einem der Ansprüche **1** bis **3,** wobei der erste Bereich (11), der dritte Bereich (13) und der fünfte Bereich (15) der Sohle (1) Stimulationsbereiche sind.

5. Sohle (1) nach einem der Ansprüche **1** bis **4,** wobei mindestens ein Stimulationsbereich ferner Stimulationselemente (3) umfasst, die in den Waben der ersten Stimulationswabenstruktur (2) verteilt sind.

6. Sohle (1) nach Anspruch **5,** wobei die Stimulationselemente (3) eine kugelförmige, halbkugelförmige, konische, parallele, polyedrische, pyramidale oder zylindrische Form aufweisen.

7. Sohle (1) nach Anspruch **5** oder Anspruch **6,** wobei der erste Bereich (11) und der fünfte Bereich (15) der Sohle (1) ferner Stimulationselemente (3) umfassen, die in den Waben ihrer ersten Stimulationswabenstruktur (2) verteilt sind, und wobei der dritte Bereich (13) der Sohle (1) eine erste leere Stimulationswabenstruktur (2) umfasst.

8. Sohle (1) nach einem der Ansprüche **1** bis **7,** wobei mindestens ein Bereich der Sohle (1) eine zweite Stimulationswabenstruktur der Fußfläche des Fußes (4) umfasst, wobei die zweite Stimulationswabenstruktur (4) eine Vielzahl von Waben umfasst, die jeweils von mindestens einer Wand begrenzt sind, die sich quer zur Fußfläche erstreckt.

9. Sohle (1) nach Anspruch **8,** wobei der mindestens eine Bereich, der die zweite Stimulationswabenstruktur (4) umfasst, aus dem zweiten Bereich (12) oder dem vierten Bereich (14) der Sohle (1) ausgewählt ist.

10. Sohle (1) nach einem der Ansprüche **8** oder **9,** wobei der zweite Bereich (12) und der vierte Bereich (14) der Sohle (1) jeweils eine zweite Stimulationswabenstruktur (4) umfassen.

11. Sohle (1) nach einem der Ansprüche **8** bis **10,** wobei die Höhe der Wände der Waben der zweiten Stimulationswabenstruktur (4) niedriger ist als die der Wände der Waben der ersten Stimulationswabenstruktur (2).

12. Sohle (1) nach einem der Ansprüche **1** bis **11,** wobei die erste Stimulationswabenstruktur (2) und/oder die zweite Stimulationswabenstruktur (4) eine Wabenstruktur aufweist.

13. Schuhelement mit einer orthopädischen Sohle (1) nach einem der Ansprüche **1** bis **12.**

14. Verfahren zur Herstellung einer orthopädischen Sohle (1) nach einem der Ansprüche **1** bis **12.**

## Claims

1. Orthopedic insole (1) comprising a plantar surface and an opposite surface, said plantar surface comprising five consecutive zones from one end of the insole (1) to the other:
- a first toe zone (11);
- a second metatarsal head zone (12);
- a third retrocapital bar zone (13);
- a fourth tarsal zone (14) comprising an anterior part and a posterior part, said anterior part comprising the anterior tarsus and said posterior part comprising a heel ring;
- a fifth posteromedial heel zone (15) delimited by the anterior part and the posterior part of the fourth zone (14);
wherein at least one of these zones is a stimulation zone comprising a first alveolar stimulation structure of the plantar surface of the foot (2), said first alveolar stimulation structure (2) comprising a plurality of alveoli, each delimited by at least one wall extending transversely to the plantar surface; and **characterized in that**
wherein at least one alveolus of the first alveolar stimulation structure (2) comprises at least one hole (5).

2. The sole (1) according to claim 1, wherein at least one stimulation zone is selected from the first zone (11), the third zone (13) or the fifth zone (15) of the sole (1).

3. The sole (1) according to claim 1 or claim 2, wherein the sole (1) comprises at least two stimulation zones selected from the first zone (11), the third zone (13) and/or the fifth zone (15) of the sole (1).

4. The sole (1) according to any of claims 1 to 3, wherein the first zone (11), the third zone (13) and the fifth zone (15) of the sole (1) are stimulation zones.

5. The sole (1) according to any of claims 1 to 4, wherein at least one stimulation zone further comprises stimulation elements (3) distributed in the alveoli of the first alveolar stimulation structure (2).

6. The sole (1) according to claim 5, wherein the stimulation elements (3) have a spherical, hemispherical, conical, parallelepiped, polyhedral, pyramidal, or cylindrical shape.

7. The sole (1) according to claim 5 or claim 6, wherein the first zone (11) and the fifth zone (15) of the sole (1) further comprise stimulation elements (3) distributed in the alveoli of their first alveolar stimulation structure (2) and wherein the third zone (13) of the sole (1) comprises an empty alveolar stimulation structure (2).

8. The sole (1) according to any of claims 1 to 7, wherein at least one area of the sole (1) comprises a second honeycomb structure for stimulating the plantar surface of the foot (4), said second alveolar stimulation structure (4) comprising a plurality of alveoli, each delimited by at least one wall extending transversely to the plantar surface.

9. The sole (1) according to claim 8, wherein the at least one area comprising said second alveolar stimulation structure (4) is selected from the second area (12) or the fourth area (14) of the sole (1).

10. The sole (1) according to any of claims 8 or 9, wherein the second zone (12) and the fourth zone (14) of the sole (1) each comprise a second alveolar stimulation structure (4).

11. The sole (1) according to any of claims 8 to 10, wherein the height of the walls of the alveoli of the second alveolar stimulation structure (4) is lower than that of the walls of the alveoli of the first alveolar stimulation structure (2).

12. The sole (1) according to any of claims 1 to 11, wherein the first alveolar stimulation structure (2) and/or the second alveolar stimulation structure (4) has a honeycomb structure.

13. Footwear element comprising an orthopedic sole (1) according to any of claims 1 to 12.

14. Method for manufacturing an orthopedic sole (1) according to any of claims 1 to 12.
